# EUROPEAN PATENT APPLICATION

(11) **EP 4 388 907 A2**
(43) Date of publication of application: **26.06.2024**
(21) Application number: 24174433.3
(22) Date of filing: 17.06.2019
(51) Int. Cl.: A24F 47/00

(54) **ATOMIZER**

(62) Divisional of application: 19933992.0
(71) Applicant: Shenzhen Smoore Technology Limited, Shenzhen, Guangdong 518102 (CN)
(72) Inventor: LEI, Guilin, Shenzhen, 518102 (CN)
(74) Representative: Herrero & Asociados, S.L.

(57) **Abstract**

An electronic atomization device and an atomizer (100) having same. The atomizer (100) comprises an atomization component (130), a liquid storage cavity (141) connected to the atomization component (130) for liquid conduction, and a gas channel communicated with the atomization component (130) for gas conduction. The atomizer (100) further comprises a gas-liquid equilibrium element (150) and a gas inlet (1430) communicated with the gas-liquid equilibrium element (150). The gas-liquid equilibrium element (150) comprises liquid storage tanks (1510, 1520) with capillary force function and a gas return groove (153). One end of the gas return groove (153) is communicated with the liquid storage cavity (141), and the other end is communicated with the gas inlet (1430). The gas return groove (153) is communicated with the liquid storage tanks (1510, 1520) such that the liquid storage tanks (1510, 1520) are communicated with the liquid storage cavity (141).

## Description

### TECHNICAL FIELD

The present application relates to the field of atomizers, and in particular, relates to an electronic atomizing device and an atomizer.

### BACKGROUND

Electronic atomizers in related art commonly have problems including: 1. liquid is prone to leak, that is, liquid media leaks, resulting in waste of liquid media, poor user experience, and even circumstances that a liquid medium contaminates electronic components and leads to failure of electronic components; 2. when an atomization speed of a liquid medium is high, unsmoothness of liquid supply occurs, so that the liquid medium cannot be quickly replenished to an atomization element, resulting in that the atomization element to dry and overheat, and thereby causing damage of the atomization element, producing burnt smell, and producing harmful substances.

### SUMMARY OF THE DISCLOSURE

Aiming at the defects existing in the aforesaid art, the present application provides a modified electronic atomizing device and atomizer.

In order to achieve the above purpose, a first aspect of the present application provides an atomizer, which comprises an atomization assembly, a liquid storage cavity fluidly connected to the atomization assembly, and an air flow passage communicated with the atomization assembly; the atomizer further comprises an air-liquid equilibrium element and an air inlet communicated with the air-liquid equilibrium element; the air-liquid equilibrium element comprises liquid storage grooves with capillary force function and an air backflow groove; the air backflow groove has one end communicated with the liquid storage cavity, and another end communicated with the air inlet; the air backflow groove is communicated with the liquid storage grooves such that the liquid storage grooves are communicated with the liquid storage cavity.

In some embodiments, the air-liquid equilibrium element comprises a surface tension isolation groove, the air backflow groove and the surface tension isolation groove are respectively disposed at two opposite sides of the air-liquid equilibrium element, and the air backflow groove is communicated with the air inlet through the surface tension isolation groove.

In some embodiments, the air-liquid equilibrium element comprises a plurality of fins disposed in parallel and at intervals, one of the liquid storage grooves is formed between evert two adjacent fins.

In some embodiments, the surface tension isolation groove and the air backflow groove laterally cut at least some of the fins, and respectively communicate corresponding liquid storage grooves with each other.

In some embodiments, the air backflow groove laterally cuts at least some of the fins along a direction being parallel to an axis of the air-liquid equilibrium element, and communicates at least some of the liquid storage grooves with the liquid storage cavity; the surface tension isolation groove laterally cuts all of the fins along a direction being parallel to an axis of the air-liquid equilibrium element, and communicates the liquid storage grooves with each other.

In some embodiments, the atomizer comprises a liquid storage casing, the air-liquid equilibrium element is filled in the liquid storage casing along an axial direction, and has an outer wall surface tightly abutting an inner surface of a side wall of the liquid storage casing.

In some embodiments, the liquid storage casing comprises a bottom wall, a space is formed between the bottom wall and the air-liquid equilibrium element, and the space forms the liquid storage cavity.

In some embodiments, the liquid storage casing comprises an opening end, and the opening end is sheathed on the atomization assembly.

In some embodiments, the fins comprise a plurality of first fins being close to the liquid storage cavity and a plurality of second fins being far away from the liquid storage cavity, first liquid storage grooves are formed between adjacent first fins, second liquid storage grooves are formed between adjacent second fins, and a width of a second liquid storage groove is larger than a width of a first liquid storage groove.

In some embodiments, the air backflow groove laterally cuts the first fins and at least some of the second fins along a direction being parallel to an axis of the air-liquid equilibrium element, and communicates the first liquid storage grooves and at least some of the second liquid storage grooves with the liquid storage cavity.

In some embodiments, the air-liquid equilibrium element further comprises a central through hole, the atomizer further comprises a liquid guide element inserting in the central through hole, the liquid guide element fluidly connects the atomization assembly with the liquid storage cavity.

In some embodiments, the air-liquid equilibrium element further comprises a through groove communicating at least some of the liquid storage grooves with the central through hole.

In some embodiments, the atomizer further comprises a base and a housing connected with the base, the atomization assembly is mounted on the base, the base comprises an atomizing cavity corresponding to the atomization assembly, the housing comprises a gap communicated with the atomizing cavity; the atomizing cavity and the gap form a part of the air flow passage.

In some embodiments, the atomization assembly comprises a porous ceramic substrate mounted on the base and a heating element disposed on the porous ceramic substrate, the porous ceramic substrate comprises a liquid absorption surface and an atomizing surface, the liquid absorption surface is connected with a lower end of the liquid guide element, the heating element is mounted on the atomizing surface, and the atomizing surface corresponds to the atomizing cavity.

In a second aspect, an electronic atomizing device is further provided, it comprises an atomizer according to any one of the above.

Advantageous effect of the present application is that: the arrangement of the air-liquid equilibrium element can balance the air pressure in the liquid storage cavity, facilitate liquid discharge, prevent dry burning, and prevent liquid leakage caused by air pressure imbalance.

### BRIEF DESCRIPTION OF THE DRAWINGS

FIG. 1 is a stereoscopic structural schematic view of an electronic atomizing device of a first embodiment of the present application.
FIG. 2 is a stereoscopic disassembled structural schematic view of the electronic atomizing device shown in FIG. 1.
FIG. 3 is a cutaway structural schematic view along the direction A-A of an atomizer in the electronic atomizing device shown in FIG. 1.
FIG. 4 is a cutaway structural schematic view along the direction B-B of an atomizer in the electronic atomizing device shown in FIG. 1.
FIG. 5 is a cutaway structural schematic view along the direction B-B of the atomizer shown in FIG. 4 after removing a housing.
FIG. 6 is a stereoscopic structural schematic view of an air-liquid equilibrium element of the atomizer shown in FIG. 3.
FIG. 7 is a stereoscopic structural schematic view from another angle of the air-liquid equilibrium element shown in FIG. 6.
FIG. 8 is a cutaway stereoscopic structural schematic view along the E-E direction of the air-liquid equilibrium element shown in FIG. 6.
FIG. 9 is a cutaway structural schematic view along the C-C direction of the air-liquid equilibrium element shown in FIG. 6 when returning air.
FIG. 10 is a cutaway structural schematic view along the C-C direction of the air-liquid equilibrium element shown in FIG. 6 when injecting liquid.
FIG. 11 is a cutaway structural schematic view along the D-D direction of the air-liquid equilibrium element shown in FIG. 6.
FIG. 12 is a cutaway structural schematic view along the E-E direction of the air-liquid equilibrium element shown in FIG. 6.
FIG. 13 is a stereoscopic structural schematic view of an atomizer of a second embodiment of the present application.
FIG. 14 is a longitudinal cutaway stereoscopic structural schematic view of the atomizer shown in FIG. 13.
FIG. 15 is a partially disassembled view of the atomizer shown in FIG. 13.
FIG. 16 is a stereoscopic structural schematic view of an air-liquid equilibrium element of the atomizer shown in FIG. 13.
FIG. 17 is a longitudinal cutaway stereoscopic structural schematic view of the air-liquid equilibrium element shown in FIG. 16.
FIG. 18 is a stereoscopic schematic view of an atomizer of an electronic atomizing device of a third embodiment of the present application.
FIG. 19 is a disassembled schematic view of a liquid storage unit and an atomization unit of the atomizer shown in FIG. 18.
FIG. 20 is a structural disassembled schematic view of the atomizer shown in FIG. 18.
FIG. 21 is a cutaway view of the atomizer shown in FIG. 18.
FIG. 22 is a partial structural schematic view of an air-liquid equilibrium element of the atomizer shown in FIG. 18.
FIG. 23 is a partial structural schematic view of another side of the air-liquid equilibrium element shown in FIG. 22.
FIG. 24 is a partial structural schematic view of an air-liquid equilibrium element of an electronic atomizing device of a fourth embodiment of the present application.
FIG. 25 is a cutaway schematic view of the air-liquid equilibrium element shown in FIG. 24.

### DETAILED DESCRIPTION

In order to describe the present application more clearly, the present application is further illustrated below in accompany with the drawings.

It should be understood that the terms "front", "rear", "left", "right", "upper", "lower", "first", "second", and so on are only for the convenience of describing technical solutions of the present application, rather than indicating that device or element referred to must have special differences, therefore they cannot be understood as any limitation of the present application. It should be noted that when one element is considered to be "connected" to another element, it can be directly connected to the other element or there may be an intermedium element at the same time. Unless otherwise defined, all technical and scientific terms used herein have the same meaning as commonly understood by those skilled in the technical field of the present application. The terms used in the description of the present application herein are only for the purpose of describing specific embodiments, and are not intended to limit the present application.

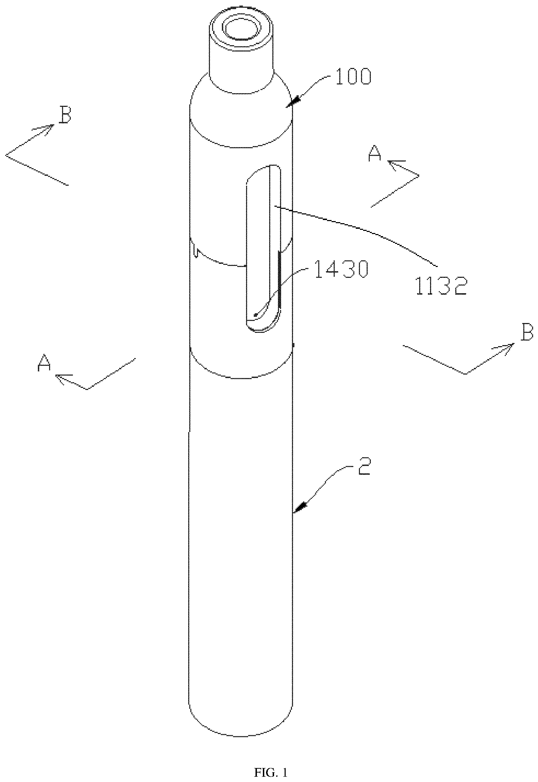

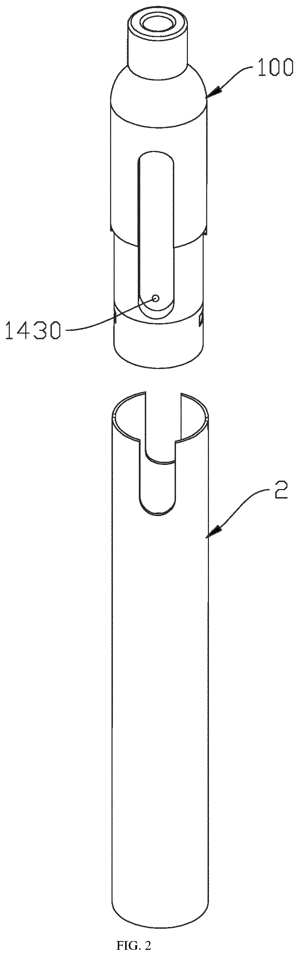

FIG. 1 and FIG. 2 show an electronic atomizing device of the first embodiment of the present application. The electronic atomizing device can be applied in atomization of liquid media, such as atomized cigarette liquid, medicine, etc., and it can include an atomizer 100 and a battery device 2 mechanically and electrically connected with the atomizer 100. The atomizer 100 is used for heating and atomizing liquid media, and the battery device 2 is used for powering the atomizer 100. Preferably, the atomizer 100 is detachably connected with the battery device 2.

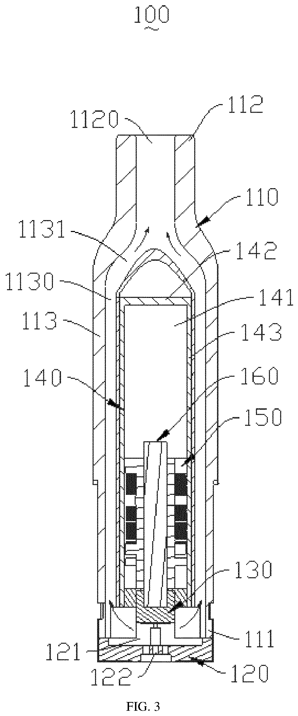

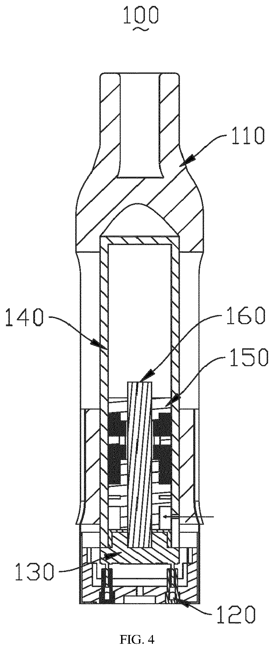

Also referring to FIG. 3 and FIG. 4, in some embodiments, the atomizer 100 can include a cylindrical housing 110, a base 120, an atomization assembly 130, a cylindrical liquid storage casing 140, an air-liquid equilibrium element 150, and a liquid guide element 160. The base 120 is disposed at an opening end of the housing 110. The atomization assembly 130 is disposed on the base 120 and located in the housing 110. The liquid storage casing 140 has one end sheathed on the atomization assembly 130, and is located in the housing 110. The air-liquid equilibrium element 150 is disposed above the atomization assembly 130, and is located in the liquid storage casing 140. The liquid guide element 160 is inserted in the air-liquid equilibrium element 150, and communicates the atomization assembly 130 with a liquid storage cavity 141 of the liquid storage casing 140.

In some embodiments, the cylindrical housing 110 can include an opening end 111 locate at a bottom thereof, a suction nozzle end 112 being opposite to the opening end 111, and a cylindrical side wall 113 connected between the opening end 111 and the suction nozzle end 112. The opening end 111 is combined with the base 120, and the suction nozzle end 112 has an air outlet 1120 that can allow a user to suck smoke by his/her mouth. The cylindrical side wall 113 encloses to form a reception cavity 1130 located at a middle part thereof, which allows components, such as the atomization assembly 130, the liquid storage casing 140, and so on, to be received therein. A gap 1131 between the side wall 113 and the liquid storage casing 140 allows air to flow through a window 1132 communicating the reception cavity 1130 with outside, the gap 1131 extends from the opening end 111 to the air outlet 1120 of the suction nozzle end 112, and the window 1132 makes the liquid storage casing 140 be at least partially exposed outside.

In some embodiments, the base 120 can include an atomizing cavity 121 located below the atomization assembly 130 and an air inlet 122 communicated with the atomizing cavity 121, the atomizing cavity 121 is communicated with the gap 1131 in the housing 110, and the air inlet 122 is communicated with the external environment. The air inlet 122, the atomizing cavity 121, the gap 1131, and the air outlet 1120 are communicated in sequence to form an air flow passage of the atomizer 100 (as shown by the arrows in FIG. 3).

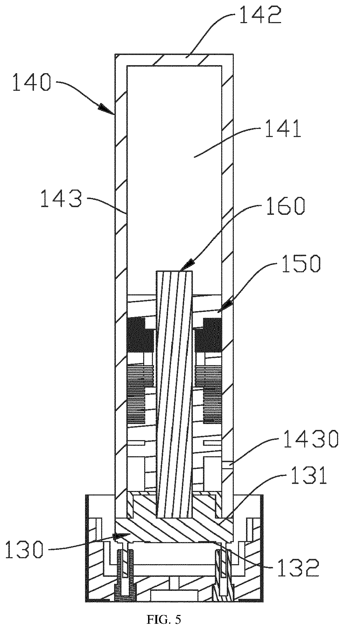

Also referring to FIG. 5, in some embodiments, the atomization assembly 130 can be mounted on the base 120, and can include a porous ceramic substrate 131 mounted on the base 120 and a heating element 132 mounted on the porous ceramic substrate 131; the porous ceramic substrate 131 includes a liquid absorption surface located at a top thereof and an atomizing surface located at a bottom thereof, the liquid absorption surface is connected with a lower end of the liquid guide element 160, the atomizing surface is exposed in the atomizing cavity 121, and the heating element 132 is mounted on the atomizing surface. Liquid in the liquid storage cavity 141 is transmitted to the liquid absorption surface via the liquid guide element 160, enters the porous ceramic substrate 131, and is heated and atomized on the atomizing surface. Atomized liquid is mixed with air in the atomizing cavity 121 and then be carried out. The atomization assembly 130 is not limited to the shape shown in the drawings, and other conventional shapes in the industry can also be adopted.

The liquid storage casing 140 can be in a cylindrical shape, and includes a bottom wall 142 and a cylindrical side wall 143 of which an end is connected with a circumferential edge of the bottom wall 142; another end of the side wall 143 forms an opening, the opening is sheathed on the atomization assembly 130. The side wall 143 defines an air entrance 1430, and the air entrance 1430 is disposed corresponding to the air-liquid equilibrium element 150.

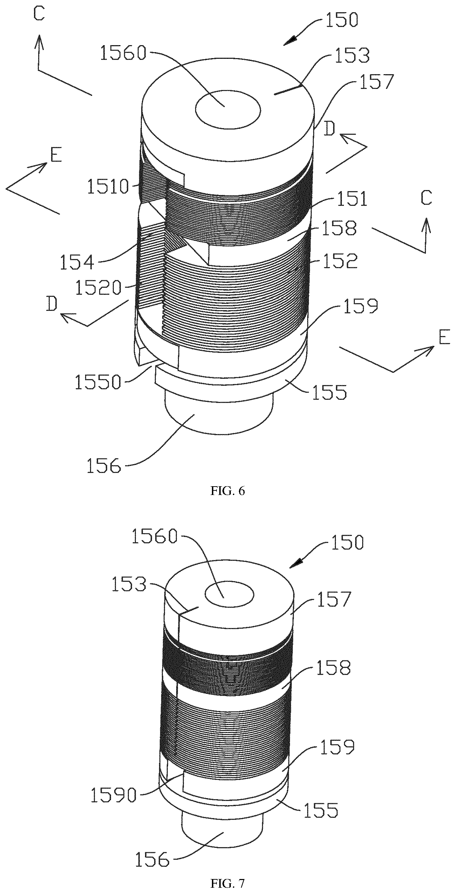

Also referring to FIG. 6 to FIG. 8, in some embodiments, the air-liquid equilibrium element 150 can be in a cylindrical shape, and can be filled in the liquid storage casing 140 along an axial direction, wherein its outer wall surface tightly abuts an inner surface of the side wall 143 of the liquid storage casing 140; that is, the liquid storage casing 140 has a part forming a reception cavity for receiving the air-liquid equilibrium element 150, and the reception cavity is communicated with the liquid storage cavity 141, such that the air-liquid equilibrium element 150 is communicated with the liquid storage cavity 141. A space is formed between the air-liquid equilibrium element 150 and the bottom wall 142 of the liquid storage casing 140, and the space forms the liquid storage cavity 141 of the liquid storage casing 140. The air-liquid equilibrium element 150 is disposed between the liquid storage cavity 141 and the atomization assembly 130, and is communicated with the air inlet 1430 of the liquid storage casing 140 so as to supply air to the liquid storage cavity 141 (as shown by the arrows in FIG. 4), and also has the function of storing liquid.

In some embodiments, the air-liquid equilibrium element 150 can include a central axle 156, and a set of first fins 151 disposed in parallel and at intervals along an axial direction and a set of second fins 152 disposed in parallel and at intervals along an axial direction, which are disposed at a periphery of the central axle 156. The first fins 151 are close to the liquid storage cavity 141, and the second fins 152 are away from the liquid storage cavity 141.

In some embodiments, the air-liquid equilibrium element 150 can further include a first isolation part 157 located at an upper portion of the central axle 156, a second isolation part 158 located at a middle portion of the central axle 156, and a third isolation part 159 located at a lower portion of the central axle 156. The firs fins 151 are disposed between the first isolation part 157 and the second isolation part 158, and the second fins 152 are disposed between the second isolation part 158 and the third isolation part 159. Each of thicknesses of the first isolation part 157, the second isolation part 158, and the third isolation part 159 is greatly larger than that of the first fin 151 and the second fin 152. In some embodiments, the air-liquid equilibrium element 150 can further include a fourth isolation part 155 located under the third isolation part 159, and a space is formed between the fourth isolation part 155 and the third isolation part 159. A top surface of the first isolation part 157 is exposed in the liquid storage cavity 141.

The central axle 156 has a central through hole 1560 for the liquid guide element 160 to pass through. A first liquid storage groove 1510 penetrating an outer circumferential surface is formed between adjacent first fins 151, and a second liquid storage groove 1520 penetrating the outer circumferential surface is formed between adjacent second fins 152. Thicknesses of the first fins 151 and the second fins 152, and widths of the first liquid storage grooves 1510 and the second liquid storage grooves 1520 are small enough to have capillary force on liquid media, so as to realize the liquid storage function. Moreover, a width of the first liquid storage groove 1510 is smaller than a width of the second liquid storage groove 1520, so that a capillary force of the first liquid storage groove 1510 is stronger. The purpose of such a configuration is that liquid flowing out through an air backflow groove 153 will first enter the first liquid storage grooves 1510, after the first liquid storage grooves 1510 is full of liquid, it is the turn of the second liquid storage grooves 1520 being far from the liquid storage cavity 141 to absorb liquid, that is, the liquid is not evenly distributed throughout the air-liquid equilibrium element 150 at the beginning, and thus the probability of liquid leakage can be reduced.

In some embodiments, thicknesses of the first fins 151 and the second fins 152 and widths of the first liquid storage grooves 1510 are ranged from 0.05mm to 0.2mm, preferably from 0.09mm to 0.15mm; width of the second liquid storage grooves 1520 are about 0.17mm. In some embodiments, the air-liquid equilibrium element 150 can further include a narrower air backflow groove 153 and a wider surface tension isolation groove 154, the air backflow groove 153 and the surface tension isolation groove 154 are respectively disposed at two opposite sides of the air-liquid equilibrium element 150, and preferably, the two form an included angle of 180 degrees. In some embodiments, a width of the air backflow groove 153 can be ranged from 0.05mm to 0.2mm, preferably from 0.09mm to 0.15mm; the air backflow groove 153 laterally cuts the first isolation part 157, the first fins 151, the second isolation part 158, and most of the second fins 152 along a direction being parallel to an axis of the air-liquid equilibrium element 150, and intersects with corresponding first light storage tanks 1510 and second light storage tanks 1520. In the air-liquid equilibrium element 150 shown in the figures, two fins 151 being close to the bottom thereof are not cut by the air backflow groove 153; the two fins 151 provide a function of enclosing the air backflow groove 153 and can increase resistance to liquid flowing downwards; if liquid leaks, it can only flow to the surface tension isolation groove 154 through the second liquid storage grooves 1520 and then leaks downwards; however, due to existence of surface tensions of the second fins 152, difficulty of such leakage will increase, so that probability of liquid leakage is reduced.

The air backflow groove 153 extends from some second fins 152 being close to a lower end to a top of the air-liquid equilibrium element 150 and is communicated with the liquid storage cavity 141, such that liquid in the liquid storage cavity 141 can flow into multiple layers of first liquid storage grooves 1510 and second liquid storage grooves 1520 through the air backflow groove 153. In some embodiments, a width of the surface tension isolation groove 154 is ranged from 1mm to 2mm, and preferably can be ranged from 1.2mm to 1.7mm; the surface tension isolation groove 154 also laterally cuts the second isolation part 158 and all of the first fins 151 and the second fins 152 along a direction being parallel to an axis of the air-liquid equilibrium element 150, and thus also intersects with corresponding first light storage tanks 1510 and second light storage tanks 1520, so as to realize tension isolation for liquid in the first liquid storage grooves 1510 and the second liquid storage grooves 1520.

The third isolation part 159 forms a first air inlet groove 1590 located at the same side as the air backflow groove 153, the first air inlet groove 1590 is communicated with the surface tension isolation groove 154 through a gap between the third isolation part 159 and the second fins 152. The fourth isolation part 155 defines a second air inlet groove 1550 located at the same side as the surface tension isolation groove 154, the second air inlet groove 1550 is communicated with the surface tension isolation groove 154 through a gap between the third isolation part 159 and the fourth isolation part 155, and the second air inlet groove 1550 is further communicated with the air inlet 1430 of the liquid storage casing 140, such that the surface tension isolation groove 154 is communicated with the air inlet 1430 of the liquid storage casing 140, and is then communicated with an outside environment through the window 1132 of the housing 110. Preferably, the air inlet 1430 is separated from the air flow passage of the atomizer 1, such that an air supply channel is separated from the air flow passage, so as to prevent adverse effect of a negative pressure formed in the air flow passage on the air supply.

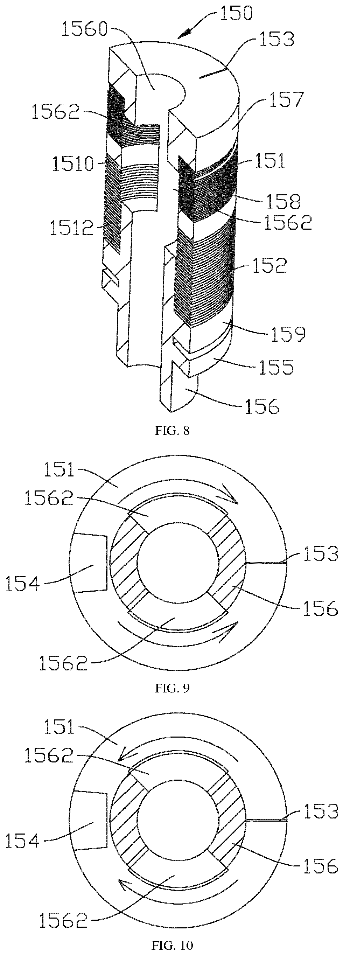

Also referring to FIG. 9 and FIG. 10, in some embodiments, backflow air with the pressure of atmosphere can enter multiple layers of liquid storage grooves 1510 via the surface tension isolation groove 154, and converges towards the air backflow groove 153 (as shown by the arrows in FIG. 9). When a negative pressure is generated in the liquid storage cavity 141, backflow air is suctioned from the air backflow groove 153; liquid in each layer of liquid storage grooves 1510, with air entering from the surface tension isolation groove 154, slowly returns towards the air backflow groove 153 and into the liquid storage cavity 141, until outside and inside pressures are balanced. When a pressure in the liquid storage cavity 141 is too high, liquid can also gradually flow downwards into multiple layers of liquid storage grooves 1510 through the air backflow groove 153 (as shown by the arrows in FIG. 10), such that the pressure in the liquid storage cavity 141 is in a balanced state. At this time, liquid leakage through the atomization assembly 130 can be avoided. In some embodiments, it is also possible to push liquid in the liquid storage grooves 1510 to return into the liquid storage cavity 141 via the air backflow groove 153 by air, so as to realize pressure balance.

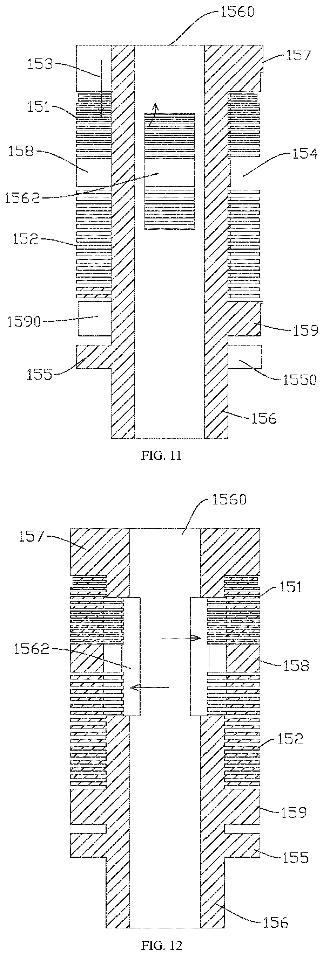

Also referring to FIG. 11 and FIG. 12, in some embodiments, the central axle 156 further includes a through slot 1562 communicating the first liquid storage grooves 1510 and the second liquid storage grooves 1520 with the central through hole 1560, such that the first liquid storage grooves 1510 and the second liquid storage grooves 1520 can perform liquid exchanged with the liquid guide element 160; that is, when liquid in the liquid guide element 160 is insufficient, liquid stored in the first liquid storage grooves 1510 and the second liquid storage grooves 1520 can enter the liquid guide element 160 via this through slot 1562 (as shown by arrows in FIG. 11), so as to maintain smooth supply of liquid. On the other hand, when liquid in the liquid guide element 160 is sufficient, and liquid in the first liquid storage grooves 1510 and the second liquid storage grooves 1520 is insufficient, liquid in the liquid guide element 160 can enter the liquid storage grooves 1510 via the through slot 1562 (as shown by arrows in FIG. 12), so as to prevent the leakage problem due to too much liquid in the liquid guide element 160 from occurring, and realize liquid balance. A width of the through slot 1562, in some embodiments, is 0.01-2mm.

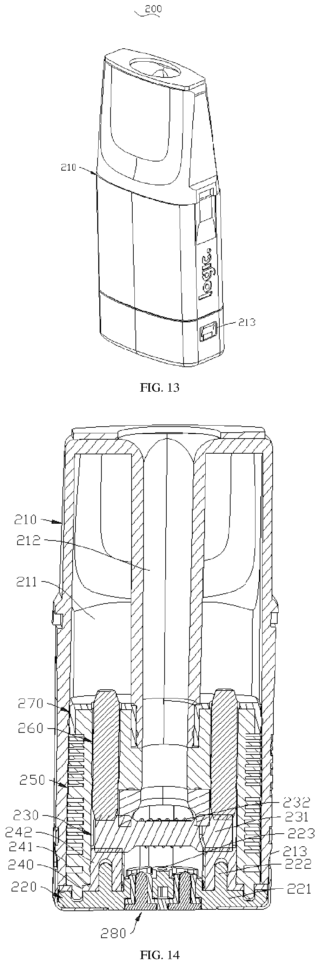

FIG. 13 and FIG. 14 show a second embodiment of an electronic atomizing device of the present application, the electronic atomizing device can be applied in fields such as electronic cigarettes, medical atomization, etc., and has advantages of smooth liquid medium supply, high safety performance, and liquid leakage resistance. The electronic atomizing device can include an atomizer 200 and a power supply device; the atomizer 200 can be used to heat and atomize liquid media, and the power supply device can be mechanically and electrically connected to the atomizer 200 to power the atomizer 200, so that atomization in the atomizer 200 is facilitated.

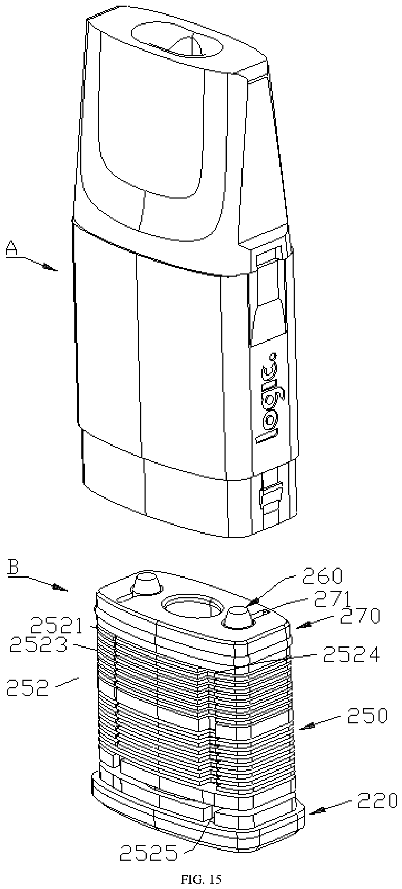

Also referring to FIG. 15, the atomizer 200 can include a liquid storage unit An and an atomization unit B; the liquid storage unit A is fluidly connected with the atomization unit B. The liquid storage unit A is used to store liquid median and allow aerosol to flow out; the atomization unit B can be used to heat and atomize liquid media.

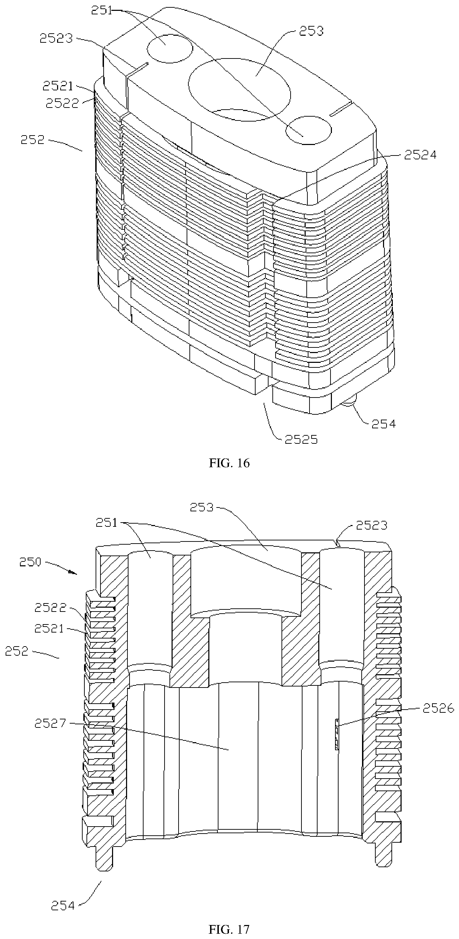

As shown in FIG. 14 to FIG. 16, the liquid storage unit A can include a housing 210; the housing 210 can be sheathed on a periphery of the atomization unit B, and its inner side can be used to form a liquid storage cavity 211 configured to receive liquid media. Specifically, space is defined in upper portions of the housing 210 and the atomization unit B, the space can form the liquid storage cavity 211. The inner side of the housing 210 further defines an air flow passage 212, the air flow passage 212 can be disposed along an axial direction of the housing 210, and can be connected with the atomization unit B for air conduction, so as to output aerosol formed by atomization in the atomization unit B. An end of the air flow passage 212 being far away from the atomization unit B defines an air outlet, the air outlet can form a cigarette nozzle for a user to suction aerosol. A closing element (not shown) can be disposed at the air outlet, so as to close the air outlet when the atomizer 210 is not used and prevent foreign substances from entering the air flow passage 212. Space is defined between the air flow passage 212 and a side wall of the housing 210 to facilitate liquid flow around the air flow passage 212. The liquid storage cavity 211 can be located at outside of the air flow passage 212. A side wall of a lower part of the housing 210 defines at least one air inlet 213; there can be two air inlets 213, which can be located at two opposite sides of the housing 210 and can allow air to enter liquid storage cavity 211.

The atomization unit B can be disposed in the housing 210, and can be located in a lower part of the liquid storage cavity 211; understandably, in some other embodiments, the atomization unit B can also be located at an outside of the housing 210, and located at a lower part of the housing 210. The atomization unit B can include a base 220, an atomization support 240, an atomization assembly 230, an air-liquid equilibrium element 250, at least two light guide elements 260, a first sealing structure 270, and an electrode component 290. The base 220 can be used to mount the atomization support 240 and the air-liquid equilibrium element 250, the housing 210 can be sheathed on the base 220, the atomization support 240 is disposed on the base 220 and can be used to support the atomization assembly 230. The atomization assembly 230 can be received in the air-liquid equilibrium element 250, and can be used to heat liquid media to form aerosol that can be suctioned by a user. The air-liquid equilibrium element 250 is disposed between the liquid storage cavity 211 and the atomization assembly 230, and can be sheathed on a periphery of the atomization assembly 230 and communicated with the air inlets 213, further communicates the liquid storage cavity 211 with outside, and thus can be used to balance an air pressure in the liquid storage cavity 211. The at least two liquid guide elements 260 can be inserted in the air-liquid equilibrium element 250, and can fluidly communicate the liquid storage cavity 211 with two ends of the atomization assembly 230, so as to provide liquid media to the atomization assembly 230. The first sealing structure 270 can be disposed between the air-liquid equilibrium element 250 and the liquid storage cavity 211, and can be used to seal a gap formed between an outer circumference of the air-liquid equilibrium element 250 and the liquid storage cavity 211. The electrode component 290 can extend out from the base 220 and electrically connected with the atomization assembly 230.

In some embodiments, the base 220 can include a base body 221, a positioning pillar 222, and an air entering channel 223; a shape and a size of the base body 221 can be adapted to a shape and a size of the opening end of the housing 210, and can be used to close the opening of the housing 210. The positioning pillar 222 can be disposed on the base body 221, and can be used to match with the atomization support 240 to position. The air entering channel 223 can be disposed in the base body 221 along an axial direction, and is disposed oppositely to the atomization assembly 230; it can allow air to enter the atomization assembly 230.

In some embodiments, the atomization support 240 can include a matching part 241 and a support part 242 disposed on the matching part 241; the matching part 241 can be located on the base body 221, and its shape and size are adapted to the base body 221; the support part 242 can be disposed to protrude towards the matching part 241, and is used to support the atomization assembly 230; the support part 242 can be sheathed on the positioning pillar 222 and matches with the positioning pillar 222 to position.

In some embodiments, the atomization assembly 230 can include an atomization core 231 and a heating element 232; the atomization core 231 may be a cotton core, which can be placed on the atomization support 240, and can be disposed in the air-liquid equilibrium element 250 along a radial direction; two ends thereof can be fluidly connected to the at least two liquid guide elements 260. The heating element 232 can be a heating wire, which can be wound around the atomization core 231, and can be electrically connected to the electrode component 290 to heat liquid medium in the atomization core 231 to form aerosol.

Also referring to FIG. 15 to FIG. 17, in some embodiments, the air-liquid equilibrium element 250 may be tube-shaped, specifically, it may be in a tubular shape with an elliptical or rectangular cross-section, and its outer circumference may be combined with an inner wall of the housing 210 in a manner of an interference fit to close the liquid storage cavity 211. The air-liquid equilibrium element 250 can be used as an atomization casing, which can accommodate the atomization assembly 230.

In some embodiments, the air-liquid equilibrium element 250 can include at least two through holes 251, a liquid storage and air exchange structure 252, and an air flow passage; the at least two through holes 251 are disposed corresponding to the at least two liquid guide elements 260, and can allow the liquid guide elements 260 to insert. In this embodiment, the at least two through holes can include two through holes 251; understandably, in some other embodiments, the at least two through holes 251 can be not limited to two through holes. The liquid storage and air exchange structure 252 can be located at outside of the two through holes 251 and can be sheathed on the periphery of the atomization assembly 230, its inner side can form an atomizing cavity 527, and it can be used to communicate the liquid storage cavity 211 with outside, so as to balance an air pressure in the liquid storage cavity 211. The air flow passage can include an air exiting channel 253; the air exiting channel 253 is communicated with the atomizing cavity 527 and located between the two through holes 251, it can allow aerosol formed in atomization by the atomization assembly 230 to output. The liquid storage and air exchange structure 252 can be further communicated with the at least two liquid guide elements 260 to balance liquid supply for the light guide elements 260.

In some embodiments, the liquid storage and air exchange structure 252 can include a plurality of fins 2521; the plurality of fins 2521 can be disposed in parallel and at intervals along an axial direction. A liquid storage groove 2522 penetrating an outer circumferential surface of the liquid storage and air exchange structure 252 can be formed between every two adjacent fins 2521; a width of the liquid storage groove 2522 is small enough to generate capillary force to liquid media, so that when liquid flows into the liquid storage groove 2522, it can form a liquid film in the liquid storage groove 2522, and then can be stored in the liquid storage groove 2522 and prevent liquid leakage. In some embodiments, a thickness of the fin 2521 and a width of the liquid storage groove 2522 are about 0.15 mm. The liquid storage groove 2522 can also be used to guide air, it can guide air entering from the air inlets 213 into the liquid storage cavity 211, so as to reduce a negative pressure formed in the liquid storage cavity 211, so that liquid in the liquid storage cavity 211 flows out smoothly.

In some embodiments, the liquid storage and air exchange structure 252 can further include at least one air backflow groove 2523; the at least one air backflow groove 2523 may include at least two air backflow grooves 2523; the at least two air backflow grooves 2523 may be disposed corresponding to the at least two through holes 251, and specifically, may include two air backflow grooves 2523. The two air backflow grooves 2523 can be disposed on the plurality of fins 2521 and can laterally cut the liquid storage grooves 2522 along a direction being parallel to an axis of the liquid storage and air exchange structure 252, and pass through to a top of the air-liquid equilibrium element 250 to communicate the liquid storage grooves 2522 with the liquid storage cavity 211; a width of the air backflow groove 2523 can be less than or equal to a width of the liquid storage groove 2522, so that liquid in the liquid storage cavity 211 can flow into each liquid storage groove 2522 through the air backflow grooves 2523. In some embodiments, a width of the air backflow groove 2523 may be ranged from 0.09mm to 0.15mm.

In some embodiments, the liquid storage and air exchange structure 252 further includes at least one surface tension isolation groove 2524; the at least one surface tension isolation groove 2524 can be disposed on the plurality of fins 2521 and laterally cut the liquid storage grooves 2522 along a direction being parallel to an axis of the liquid storage and air exchange structure 252; it can be used to realize tension isolation for liquid in these liquid storage grooves 2522. In some embodiments, the at least one surface tension isolation groove 2524 may include at least two surface tension isolation grooves 2524 disposed corresponding to the at least two through holes 251; specifically, it may include two surface tension isolation grooves 2524, the two surface tension isolation grooves 2524 can be disposed corresponding to the two air backflow grooves 2523, and are respectively located on two opposite sides of the through hole 251 with the air backflow grooves 2523 and located at a position at 180 degrees; both of them laterally cut the liquid storage grooves 2522 along a direction being parallel to an axis of the liquid storage and air exchange structure 252, so that tension of liquid in each liquid storage groove 2522 is isolated.

In some embodiments, backflow air at the atmospheric pressure can enter multiple layers of the liquid storage grooves 2522 through the surface tension isolation groove 2524, and converges toward the air backflow grooves 2523. When the liquid storage cavity 211 generates a negative pressure, it can only suction aerosol from the air backflow grooves 2523; air entering the air inlets 213 can enter each layer of the liquid storage grooves 2522 from the surface tension isolation groove 2524, and slowly flows into the liquid storage cavity 211 from the air backflow grooves 2523 until air-liquid equilibrium is reached. When the air pressure in the liquid storage cavity 211 is balanced, liquid can also gradually flow downwards into multiple layers of the liquid storage grooves 2522 via the air backflow grooves 2523. At this time, liquid leakage through the atomization assembly 211 can be avoided. In some embodiments, a width of the surface tension isolation groove 2524 is ranged from 1.2 mm to 1.7 mm.

In some embodiments, the liquid storage and air exchange structure 252 further includes an air entering groove 2525, the air entering groove 2525 can be disposed at a lower part of the surface tension isolation groove 2524, and can be disposed to stagger with the air backflow grooves 2523; it can be a wide groove and is communicated with the air inlets 213, and can allow air to enter the surface tension isolation groove 2524.

In some embodiments, the liquid storage and air exchange structure 252 further includes at least one through groove 2526; the at least one through groove 2526 may be one or more; in some embodiments, the at least one through groove 2526 and the air backflow grooves 2523 are provided correspondingly, there can be two through grooves 2526, which can be used to communicate the through holes 251 with the liquid storage grooves 2522, so that the liquid storage grooves 2522 can exchange liquid with the liquid guide elements 260; that is, when liquid in the liquid guide elements 260 is insufficient, liquid stored in the liquid storage grooves 2522 can enter the liquid guide elements 260 through the through grooves 2526, so as to maintain smooth supply of liquid and prevent the atomization core 231 from drying out. Conversely, when liquid in the liquid guide elements 260 is sufficient and liquid in the liquid storage grooves 2522 is insufficient, liquid in the liquid guide elements 260 can flow back into the liquid storage grooves 2522 via the through grooves 2526. In some embodiments, a width of the through groove 2526 may be ranged from 0.01 mm to 2 mm.

In some embodiments, the liquid storage and air exchange structure 252 further includes at least one isolation portion 2528; the isolation portion 2528 can be disposed between the plurality of fins 2521, and the at least one isolation portion 2528 may further be provided with one or more isolation portion(s) 2528, which can divide the plurality of fins 2521 into at least two sections of liquid storage and air exchange units arranged along an axial direction. In this embodiment, there may be one isolation portion 2528, which can divide the plurality of fins 2521 into two sections of liquid storage and air exchange units. When the liquid storage grooves 2522 in a liquid storage and air exchange unit near one end of the liquid storage cavity 211 are full of liquid, liquid can enter the next section of liquid storage and air exchange unit in sequence. A cut surface 25281 may be disposed on the isolation portion 2528; the cut surface 25281 may be located on one side of the surface tension isolation groove 2524 to facilitate flow of air and liquid. In some embodiments, a width of a liquid storage groove 2522 in the liquid storage and air exchange unit being close to the liquid storage cavity 211 is larger than a width of a liquid storage groove 2522 being far away from the liquid storage cavity 211, so that liquid leakage can be prevented.

In some embodiments, the air-liquid equilibrium element 250 can further include a positioning structure 254; the positioning structure 254 can be a positioning pillar, which can be disposed at an end of the liquid storage and air exchange structure 252 being far away from the liquid storage cavity 211, and can be used to mount and position the air-liquid equilibrium element 250.

In some embodiments, the at least two liquid guide elements 260 are disposed corresponding to the at least two through holes 251, and may include two liquid guide elements 260, which may be correspondingly inserted in the through holes 251, and located at two ends of the atomization core 231 and fluidly communicated to the atomization core 231. The liquid guide elements 260 may be cotton cores. Understandably, in some other embodiments, the liquid guide elements 260 may not be limited to cotton cores.

In some embodiments, the first sealing structure 270 can be a sealing sleeve, which can be sheathed on the air-liquid equilibrium element 250, and can define an avoidance hole corresponding to the liquid guide elements 260, the air backflow grooves 2523, and the air outlet 253 thereon. The first sealing structure 270 can be a silicone sleeve or a rubber sleeve.

In some embodiments, the electrode component 290 can include two electrode pillars, the two electrode pillars are respectively a positive electrode pillar and a negative electrode pillar, which are disposed abreast on the base body 221, and are respectively located at two sides of the air entering channel 223; an end thereof inserting in the base 220 can be electrically connected with the heating body 232 of the atomization assembly 230 by arranging wires, and another end thereof can be electrically connected with the power supply device.

FIGS. 15 to 17 further show some preferred embodiments of an atomization casing of the present application. The atomization casing of the present application forms the air-liquid equilibrium element 250 of the present application. The atomization casing can include a main body; an inside of the main body can form the atomizing cavity 2527; the main body can be cylindrical, and it can include at least one through hole 251, the liquid storage and air exchange structure 252, and an air flow channel; the at least one through hole 251 can be arranged along a longitudinal direction, and can be used to mount the liquid guiding elements 260. In some embodiments, the at least one through hole 251 can include at least two through holes 251, and the at least two through holes 251 may be disposed at both sides of the air flow channel. Understandably, in some other embodiments, the number of the through holes 251 may not be limited to two. The liquid storage and air exchange structure 252 can be disposed on the periphery of the at least one through hole 251, and its specific structure has been described above and will not be repeated here. The air flow channel can be defined in the main body, and it can be communicated with the atomizing cavity 2527 and can be used for output of aerosol in the atomizing cavity 2527.

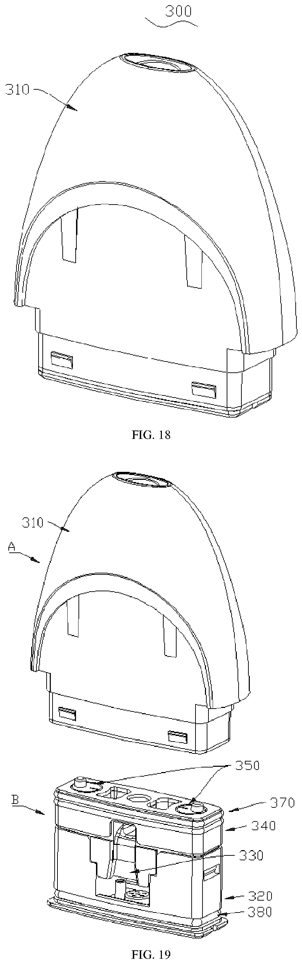

FIG. 18 and FIG. 19 show a third embodiment of an electronic atomizing device of the present application, the electronic atomizing device can be applied in fields such as electronic cigarettes, medical atomization, etc., and has advantages of smooth liquid medium supply, high safety performance, and liquid leakage resistance.

The electronic atomizing device can include an atomizer 300 and a power supply device; the power supply device can be electrically connected with the atomizer 300 to power the atomizer 300, so that atomization in the atomizer 300 is facilitated.

As shown in FIG, 18 and FIG. 19, in some embodiments, the atomizer 300 can include a liquid storage unit An and an atomization unit B; the liquid storage unit A is fluidly connected with the atomization unit B. The liquid storage unit A is used to store liquid media and allow aerosol to flow out; the atomization unit B can be used to heat and atomize liquid media.

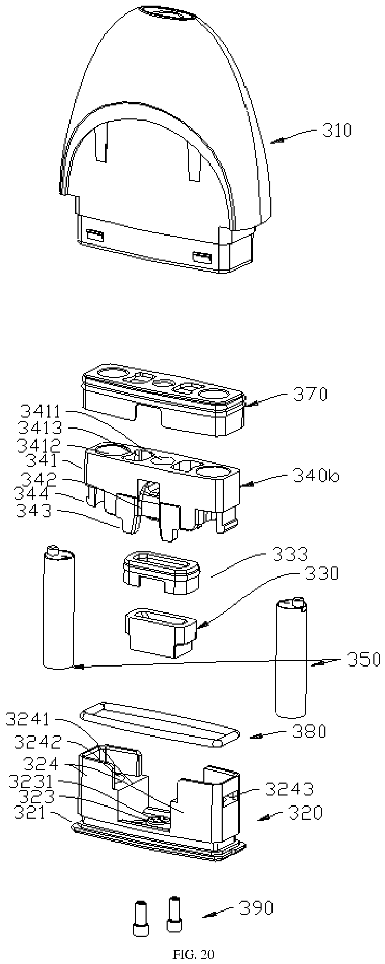

As shown in FIG. 19 and FIG. 20, the liquid storage unit A can include a housing 310; the housing 310 can be sheathed on a periphery of the atomization unit B, and its inner side can be used to form a liquid storage cavity 311 configured to receive liquid media. Specifically, space is defined in upper portions of the housing 310 and the atomization unit B, the space can form the liquid storage cavity 311. The inner side of the housing 310 further defines an air flow passage 312, the air flow passage 312 can be disposed along an axial direction of the housing 310, and can be connected with the atomization unit B for air conduction, so as to output aerosol formed by atomization in the atomization unit B. An end of the air flow passage 312 being far away from the atomization unit B defines an air outlet, the air outlet can form a cigarette nozzle for a user to suction aerosol. A closing element can be disposed at the air outlet, so as to close the air outlet when the atomizer 310 is not used and prevent foreign substances from entering the air flow passage 312. Space is defined between the air flow passage 312 and a side wall of the housing 310 to facilitate liquid flow around the air flow passage 312. The liquid storage cavity 311 can be located at outside of the air flow passage 312.

In some embodiments, the atomization unit B can be disposed in the housing 310; understandably, in some other embodiments, the atomization unit B can also be located at an outside of the housing 310, and located at a lower part of the housing 310. The atomization unit B can include a base 320, an atomization assembly 330, an atomization casing 340, and at least one air-liquid equilibrium element 350. The base 320 can be used to mount the atomization assembly 330, the atomization casing 340, and the air-liquid equilibrium element 350, the housing 310 can be sheathed on the base 320. The atomization assembly 330 is mounted on the base 320 and received in the atomization casing 340, and can be used to heat liquid media to form aerosol that can be suctioned by a user. The atomization casing 340 is disposed on the base 320, one end thereof can be inserted in the base 320 and detachably connected with the base 320; it can be used to match with the base 320 to mount the atomization assembly 330. The at least one air-liquid equilibrium element 350 can include two air-liquid equilibrium elements. The two air-liquid equilibrium elements can be respectively located at a first side and a second side of the atomization assembly 330, and are mounted in the base 320 and inserted in the atomization casing 340, and extend towards the direction of the housing 310; they can be disposed in a lower part of the liquid storage cavity 311 and communicated with the liquid storage cavity 311, and can be used to adjust air-liquid balance in the liquid storage cavity 311. In some embodiments, the first side and the second side of the atomization assembly 330 can be two opposite sides of the atomization assembly 330.

In some embodiments, the atomization unit B can further include a first sealing structure 370, a second sealing structure 380, and an electrode component 390. The first sealing structure 270 can be disposed between the atomization casing 340 and the liquid storage cavity 311, and can be used to seal a gap formed between the housing 310 and the atomization unit B to prevent liquid leakage. The second sealing structure 380 can be sheathed on the base 320 and can sealingly connect the housing 310 and the base 320. The electrode component 390 can extend out from the base 320 and is electrically connected with the atomization assembly 330.

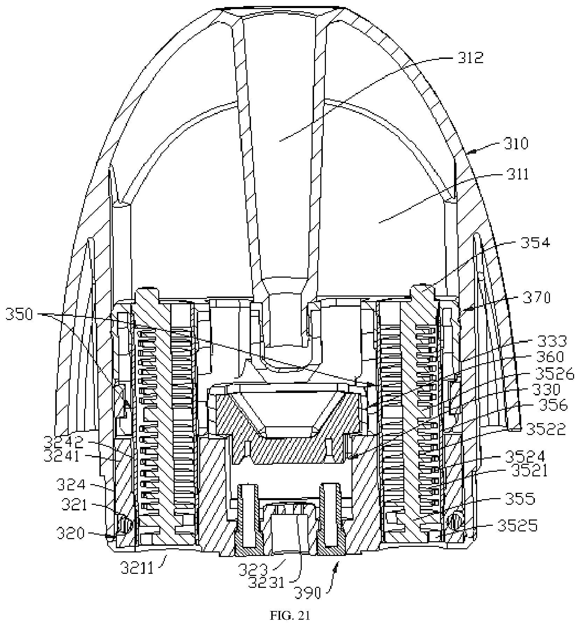

Furthermore, as shown in FIG. 20 and FIG. 21, the base 320 can include a base body 321 and two mounting seats 324. A shape and a size of the base body 321 can be adapted to a shape and a size of an opening end of the housing 310, and it can be used to close an opening of the housing 310. The two mounting seats 324 are disposed separately, they can be used to support the atomization assembly 330, and can be used to mount the air-liquid equilibrium element 350.

The base 320 can be provided thereon with at least one air inlet 3211; the at least one air inlet 3211 can include two air inlets 3211; the two air inlets 3211 can be located at a bottom of the base body 321, and respectively located at both sides of a central axis of the base body 321. The base 320 can be further provided thereon with an air entering channel 323; the air entering channel 323 is provided at the bottom of the base body 321 and located between the two air inlets 3211, and is arranged along an axial direction to be communicated with the atomization assembly 330 and thereby allow air to enter the atomization assembly 330. The two air inlets 3211 can be located at two opposite sides of the air entering channel 323, so as to prevent the air-liquid equilibrium element 350 from communicating with the air entering channel 323, and thereby avoiding a negative pressure generated by the air flow passage from causing leakage of liquid medium when aerosol is suction. Of course, understandably, in some other embodiments, when there is one air inlet 3211, it may be located at one side of the air entering channel 323. A layer of mesh body 3231 can be disposed in the air entering channel 323; the mesh body 3231 can be integrally formed with the base 320. Since diameters of meshes are small, liquid medium can form a layer of liquid film in each mesh, thereby preventing leakage of the liquid medium.

Each mounting seat 324 can include a boss 3241 and a mounting hole 3242 defined in the boss 3241. The boss 3241 can cooperate with the boss 3241 of the other mounting seat 324 to support the atomization assembly 330, and the space between the two bosses 3241 can form an atomizing cavity communicating with the air entering channel 323. The mounting hole 3242 is disposed corresponding to the air inlet 3211 and communicates with the air entering channel 323. The mounting hole 3242 is disposed along an axial direction, and can allow the air-liquid equilibrium element 350 to be inserted and mounted in the base 320. A side wall of an outer circumference of the boss 3241 extends towards a direction of the liquid storage cavity 311, and a side wall thereof being opposite to the atomizing cavity can be provided with a buckle 243 to cooperate with the outer atomization housing 340 to mount.

In some embodiments, the atomization assembly 330 can be placed on the bosses 3241 of the two mounting seats 324, and it can abut against the bosses 3241 respectively. The atomization assembly 330 includes a porous ceramic substrate and a heating element; the porous ceramic substrate can be disposed to be opposite to the base 320, and can be used for liquid absorption. The heating element can be disposed on the porous ceramic substrate, and can be used to heat e liquid medium in the porous ceramic substrate to form aerosol.

Furthermore, as shown in FIGS. 19-21, in some embodiments, an elastic member 333 is sheathed on the porous ceramic substrate; the elastic member 333 has one end abutting against a top wall of a cover body 342 of the atomization housing 340 and another end against the porous ceramic substrate; it can be used to prevent the porous ceramic substrate from being crushed, and at the same time, it can also play a buffering role. The elastic member 333 can be a silicone sleeve or a rubber sleeve; understandably, in some other embodiments, the elastic member 333 may not be limited to a silicone sleeve or a rubber sleeve; and in some other embodiments, it may also be omitted.

In some embodiments, the atomization housing 340 can include a sleeve body 341, a cover body 342, positioning portions 343, and a buckle 344. The sleeve body 341 can be sheathed on the periphery of the air-liquid equilibrium element 350, and is provided thereon with an air outlet 3411; the air outlet 3411 is communicated with the atomizing cavity and the air flow passage 312 to allow aerosol to output. The atomization housing 340 can be provided with at least two lower liquid holes 3412; the at least two lower liquid holes 3412 can be defined in the sleeve body 341, and are located at both sides of the air outlet 3411; specifically, they are located at a first side and a second side of the atomization assembly 330 and fluidly connected with the atomization assembly 330, so as to supply liquid medium to the atomization assembly 330. The sleeve body 341 is further provided with through holes 3413, the number and positions of the through holes 3413 correspond to the air-liquid equilibrium element 350; they are located at the first side and the second side of the atomization assembly 330 and can allow the air-liquid equilibrium element 350 to extend out. The cover body 342 is disposed inside the cover body 341, it is located at a lower part of the air outlet 3411, and is spaced from the air outlet 3411 to form a through slot penetrating two opposite sides of the cover body 341. The through slot is communicated with the air outlet 3411 to facilitate aerosol output. The inside of the cover 342 can form a receiving space for receiving the atomization assembly 330. The positioning portions 343 are disposed on the sleeve body 341, they can be in two sets, the two sets of positioning portions can be located on two opposite sides in a long axis direction of the sleeve body 341, and each set of positioning portions can include two positioning portions 343. The two positioning portions 343 are arranged at intervals and are respectively located at two sides of the cover body 342, they extend toward the base 320 to abut against the bosses 3241. The buckles 344 can be disposed at two opposite sides in a short axis direction of the sleeve body 341, and can extend toward the base 320 to be buckled in buckle holes 3243 of the base 320.

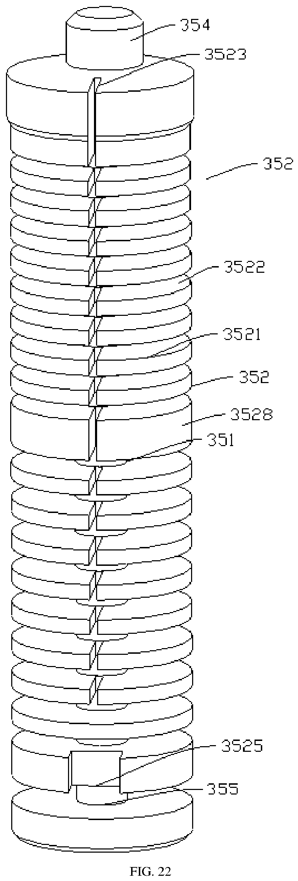

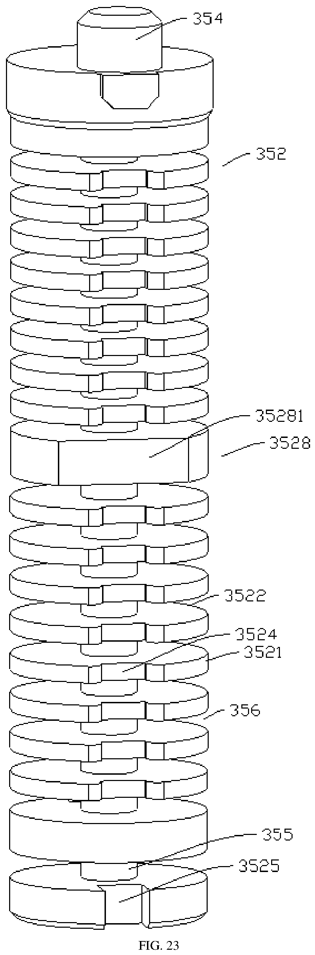

As shown in FIGS. 20-23, in some embodiments, an overall height of each air-liquid equilibrium element 350 can be adapted to an overall height of the atomization unit B. The two air-liquid equilibrium elements 350 can be respectively located at sides of the two lower liquid holes 3412 being opposite to the air outlet 3411, and are used to balance air and liquid in the liquid storage cavity 311, thereby reducing a negative pressure in the liquid storage cavity 311 and enabling air to flow smoothly from the lower liquid holes 3412 to the atomization assembly 330, so as to prevent the atomization assembly 330 from being damaged due to dry burning and overheating, and to avoid generation of burnt smell and harmful substances. In addition, it can store liquid to prevent liquid leakage.

In some embodiments, each air-liquid equilibrium element 350 can include a column body 351 and a liquid storage and air exchange structure 352 disposed on the periphery of the column body. The column body 351 can be in a longitudinal shape, and can be used to mount the liquid storage and air exchange structure 352. The liquid storage and air exchange structure 352 can be communicated to the liquid storage cavity 311, and can be used to adjust air-liquid balance in the liquid storage cavity 311.

In some embodiments, the liquid storage and air exchange structure 352 can include a plurality of fins 3521; the plurality of fins 3521 can be disposed in parallel and at intervals along an axial direction. A liquid storage groove 3522 penetrating an outer circumferential surface of the liquid storage and air exchange structure 352 can be formed between every two adjacent fins 3521; a width of the liquid storage groove 3522 is small enough to generate capillary force to liquid media, so that when liquid flows into the liquid storage groove 3522, it can form a liquid film in the liquid storage groove 3522, and then can be stored in the liquid storage groove 3522 and prevent liquid leakage. In some embodiments, a thickness of the fin 3521 and a width of the liquid storage groove 3522 are about 0.15 mm. The liquid storage groove 3522 can also be used to guide air, it can guide air entering from the air inlets 3211 into the liquid storage cavity 311, so as to reduce a negative pressure formed in the liquid storage cavity 311, so that air in the liquid storage cavity 311 flows out smoothly.

In some embodiments, the liquid storage and air exchange structure 352 can further include an air backflow groove 3523; the air backflow groove 3523 can be disposed on the plurality of fins 3521 and can laterally cut the liquid storage grooves 3522 along a direction being parallel to an axis of the liquid storage and air exchange structure 352, and pass through to a top of the air-liquid equilibrium element 350 to communicate the liquid storage grooves 3522 with the liquid storage cavity 311; a width of the air backflow groove 3523 can be less than or equal to a width of the liquid storage groove 3522, so that liquid in the liquid storage cavity 311 can flow into each liquid storage groove 3522 through the air backflow grooves 3523. In some embodiments, a width of the air backflow groove 3523 may be ranged from 0.09mm to 0.15mm.

In some embodiments, the liquid storage and air exchange structure 352 further includes a surface tension isolation groove 3524; the surface tension isolation groove 3524 can be disposed on the plurality of fins 3521 and laterally cut the liquid storage grooves 3522 along a direction being parallel to an axis of the liquid storage and air exchange structure 352; it can be used to realize tension isolation for liquid in these liquid storage grooves 3522. In some embodiments, the surface tension isolation groove 3524 and the air backflow groove 3523 can be respectively located on two opposite sides of the column body 351 with and located at a position at 180 degrees; both of them laterally cut the liquid storage grooves 3522 along a direction being parallel to an axis of the liquid storage and air exchange structure 352, so that tension of liquid in each liquid storage groove 3522 is isolated.

In some embodiments, backflow air at the atmospheric pressure can enter multiple layers of the liquid storage grooves 3522 through the surface tension isolation groove 3524, and converges toward the air backflow groove 3523. When the liquid storage cavity 311 generates a negative pressure, it can only suction aerosol from the air backflow groove 3523; air entering the air inlets 3211 can enter each layer of the liquid storage grooves 3522 from the surface tension isolation groove 3524, and slowly flows into the liquid storage cavity 311 from the air backflow groove 3523 until air-liquid equilibrium is reached. When the air pressure in the liquid storage cavity 311 is balanced, liquid can also gradually flow downwards into multiple layers of the liquid storage grooves 3522 via the air backflow groove 3523. At this time, liquid leakage through the atomization assembly 11 can be avoided. In some embodiments, a width of the surface tension isolation groove 3524 is ranged from 1.2 mm to 1.7 mm.

In some embodiments, the liquid storage and air exchange structure 352 further includes an air entering groove 3525, the air entering groove 3525 can be disposed at a lower part of the surface tension isolation groove 3524, and can be disposed to stagger with the air backflow groove 3523; it can be a wide groove and is communicated with the air inlets 3211, and can allow air to enter the surface tension isolation groove 3524.

In some embodiments, the liquid storage and air exchange structure 352 further includes at least one isolation portion 3528; the isolation portion 3528 can be disposed between the plurality of fins 3521, and the at least one isolation portion 3528 may further be provided with one or more isolation portion(s) 3528, which can divide the plurality of fins 3521 into at least two sections of liquid storage and air exchange units arranged along an axial direction. In this embodiment, there may be one isolation portion 3528, which can divide the plurality of fins 3521 into two sections of liquid storage and air exchange units. When the liquid storage grooves in a liquid storage and air exchange unit near one end of the liquid storage cavity 311 are full of liquid, liquid can enter the next section of liquid storage and air exchange unit in sequence. A cut surface 35281 may be disposed on the isolation portion 3528; the cut surface 35281 may be located on one side of the surface tension isolation groove 3524 to facilitate flow of air and liquid. In some embodiments, a width of a liquid storage groove 3522 in the liquid storage and air exchange unit being close to the liquid storage cavity is larger than a width of a liquid storage groove 3522 being far away from the liquid storage cavity 311, so that liquid leakage can be prevented.

In some embodiments, the air-liquid equilibrium element 350 further includes a positioning structure 354; the positioning structure 354 can be disposed at one end of the column body 351, and can be used for mounting and positioning the air-liquid equilibrium element 350 to avoid a direction of the air-liquid equilibrium element 350 being reversed.

In some embodiments, the air-liquid equilibrium element 350 further includes a sleeve barrel 356; the sleeve barrel 356 can be sheathed on the periphery of the column body 351, specifically, it can be sheathed on the outside of the fins 3521; it can avoid liquid from leaking into the atomizing cavity, and avoid aerosol in the atomizing cavity from entering the liquid storage grooves 3522.

In some embodiments, the first sealing structure 370 can be a sealing sleeve; it can be sheathed on the atomization housing 340, and is provided thereon with an avoidance hole corresponding to the lower liquid hole 3412, the air outlet 3411, and the through hole 3413. The positioning structure 3542 of the air-liquid equilibrium element 350 can be configured to extend out of the sealing sleeve. The first sealing structure 370 can be a silicone sleeve or a rubber sleeve.

In some embodiments, the second sealing structure 380 can be a sealing ring, which can be sheathed on the base body 321, and it can be a rubber ring or a silicone ring, which can be used to seal a gap between the base body 321 and the housing 310.

In some embodiments, the electrode component 390 can include two electrode pillars, the two electrode pillars are respectively a positive electrode pillar and a negative electrode pillar, which are disposed abreast on the base body 321, and are respectively located between the air entering channel 3212 and the air inlet 3211; an end thereof inserting in the base 320 can be electrically connected with the atomization assembly 330 by arranging wires, and another end thereof can be electrically connected with the power supply device.

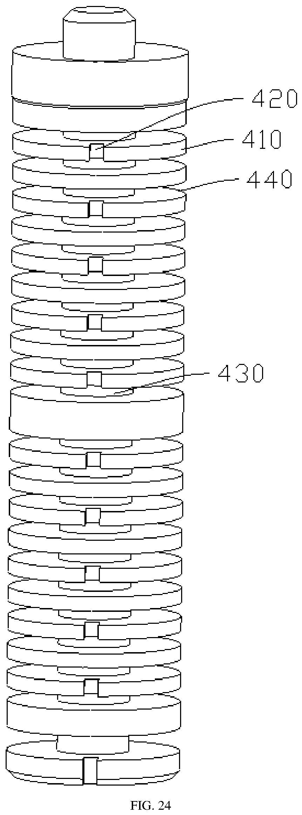

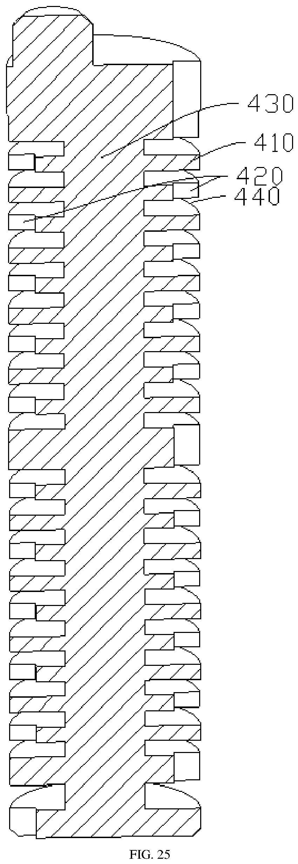

FIG. 24 and FIG. 25 show a fourth embodiment of the electronic atomizing device of the present application, which differs from the third embodiment in that the surface tension partition groove can be omitted. An air backflow groove 420 can include two sets of air backflow groove units 420; the two sets of air return groove units 420 can be disposed at two opposite sides of a column body 430 and arranged in an angle of 180 degrees. The air backflow groove units 420 in each set of air backflow groove units 420 are alternately arranged with the air backflow groove units 420 of the other set of air backflow groove units 420, and are arranged in an angle of 180 degrees. Each air backflow groove unit 420 can be provided on a fin 410, and can be disposed along a radial direction of the fin 410 to communicate two adjacent liquid storage grooves 440; one group of air backflow groove units 420 can be located in the same straight linear direction, and two return groove units 420 adjacently arranged on the same straight line can be separated by a fin 410. It is understandable that in some other embodiments, the multiple air backflow groove units 420 may not be limited to be located on the same straight line, and they may also be staggered.

It is understandable that the above embodiments only express preferred implementations of the present application. The description is specific and detailed, but should not be accordingly construed as any limitation to the patent scope of the present application. It should be pointed out that for those of ordinary skill in the art, the above technical features can be freely combined and various modifications and improvements can be made without departing from the concept of the present application, these all belong to the protection scope of the present application; therefore, any equivalent changes and modifications made according to the scope of claims of the present application shall fall within the scope of the claims of the present application.

## Claims

1. An atomizer (100, 200, 300), **characterized by** comprising a base (120, 220, 320), an atomization assembly (130, 230, 330), a liquid storage cavity (141, 211, 311) fluidly connected to the atomization assembly (130, 230, 330), and an air flow passage communicated with the atomization assembly (130, 230, 330); wherein, the atomizer (100, 200, 300) further comprises an air-liquid equilibrium element (150, 250, 350) and an air inlet (1430 ,213, 3211) communicated with the air-liquid equilibrium element (150, 250, 350);
the air-liquid equilibrium element (150, 250, 350) is arranged on the base (120, 220, 320), an atomizing cavity (121, 2527) is defined in an inner side of the air-liquid equilibrium element (150, 250, 350), the atomization assembly (130, 230, 330) is received in the atomizing cavity (121, 2527), an through hole (251) is defined in the air-liquid equilibrium element (150, 250, 350) and extends from a top of the air-liquid equilibrium element (150, 250, 350) to a bottom of the air-liquid equilibrium element (150, 250, 350), the through hole (251) communicates the liquid storage cavity (141, 211, 311) with the atomizing cavity (121, 2527); the air-liquid equilibrium element (150, 250, 350) is configured to supply air to the liquid storage cavity (141, 211, 311) and store liquid, and the air-liquid equilibrium element (150, 250, 350) comprises a plurality of liquid storage grooves (1510, 1520, 2522, 3522, 440) with capillary force function and an air backflow groove (153, 2523, 3523, 420); the air backflow groove (153, 2523, 3523, 420) has one end communicated with the liquid storage cavity (141, 211, 311), and another end communicated with the air inlet (1430, 213, 3211);
the air backflow groove (153, 2523, 3523, 420) is communicated with the plurality of liquid storage grooves (1510, 1520, 2522, 3522, 440) such that the plurality of liquid storage grooves (1510, 1520, 2522, 3522, 440) are communicated with the liquid storage cavity (141, 211, 311);
along an axis of the atomizer (100, 200, 300), a position of the atomization assembly (130, 230, 330) is higher than at least some of the plurality of liquid storage grooves (1510, 1520, 2522, 3522, 440).

2. The atomizer (100, 200, 300) according to claim 1, wherein the atomizer (100, 200, 300) comprises a first sealing structure (270, 370), the first sealing structure (270, 370) is arranged between the air-liquid equilibrium element (150, 250, 350) and the liquid storage cavity (141, 211, 311), and a hole corresponding to the through hole (251) is defined in the first sealing structure (270, 370).

3. The atomizer (100, 200, 300) according to claim 1, wherein the plurality of liquid storage grooves (1510, 1520, 2522, 3522, 440) are located on a periphery of the atomization assembly (130, 230, 330).

4. The atomizer (100, 200, 300) according to claim 1, wherein at least a part of the air backflow groove (153, 2523, 3523, 420) extends along a central axis of the atomizer (100, 200, 300).

5. The atomizer (100, 200, 300) according to claim 1, wherein the atomization assembly (130, 230, 330) comprises a porous ceramic substrate (131).

6. The atomizer (100, 200, 300) according to claim 1, wherein the air-liquid equilibrium element (150, 250, 350) comprises a surface tension isolation groove (154, 2524, 3524), the surface tension isolation groove (154, 2524, 3524) is configured to isolate tension for liquid in the plurality of liquid storage grooves (1510, 1520, 2522, 3522, 440), the air backflow groove (153, 2523, 3523, 420) and the surface tension isolation groove (154, 2524, 3524) are respectively disposed at two opposite sides of the air-liquid equilibrium element (150, 250, 350), and the air backflow groove (153, 2523, 3523, 420) is communicated with the air inlet (1430, 213, 3211) through the surface tension isolation groove (154, 2524, 3524).

7. The atomizer (100, 200, 300) according to claim 6, wherein the air-liquid equilibrium element (150, 250, 350) comprises a plurality of fins (151, 152, 2521, 3521, 410) disposed in parallel and at intervals, one of the plurality of liquid storage grooves (1510, 1520, 2522, 3522, 440) is formed between evert two adjacent fins (151, 152, 2521, 3521, 410).

8. The atomizer (100, 200, 300) according to claim 7, wherein the surface tension isolation groove (154, 2524, 3524) and the air backflow groove (153, 2523, 3523, 420) laterally cut at least some of the fins (151, 152, 2521, 3521, 410), and respectively communicate corresponding liquid storage grooves (1510, 1520, 2522, 3522, 440) with each other; and
wherein the air backflow groove (153, 2523, 3523, 420) laterally cuts at least some of the fins (151, 152, 2521, 3521, 410) along a direction being parallel to an axis of the air-liquid equilibrium element (150, 250, 350), and communicates at least some of the plurality of liquid storage grooves (1510, 1520, 2522, 3522, 440) with the liquid storage cavity (141, 211, 311); the surface tension isolation groove (154, 2524, 3524) laterally cuts all of the fins (151, 152, 2521, 3521, 410) along a direction being parallel to an axis of the air-liquid equilibrium element (150, 250, 350), and communicates the plurality of liquid storage grooves (1510, 1520, 2522, 3522, 440) with each other.

9. The atomizer (100) according to any one of claims 1-8, wherein the atomizer (100) comprises a liquid storage casing (140), the air-liquid equilibrium element (150) is filled in the liquid storage casing (140) along an axial direction, and has an outer wall surface tightly abutting an inner surface of a side wall of the liquid storage casing (140).

10. The atomizer (100) according to claim 9, wherein the liquid storage casing (140) comprises a bottom wall, a space is formed between the bottom wall and the air-liquid equilibrium element, and the space forms the liquid storage cavity (141); or
wherein the liquid storage casing (140) comprises an opening end, and the opening end is sheathed on the atomization assembly.

11. The atomizer (100) according to any one of claims 7-8, wherein the fins comprise (151, 152) a plurality of first fins (151) being close to the liquid storage cavity (141) and a plurality of second fins (152) being far away from the liquid storage cavity (141), first liquid storage grooves (1510) are formed between adjacent first fins (151), second liquid storage grooves (1520) are formed between adjacent second fins (152), and a width of a second liquid storage groove (1520) is larger than a width of a first liquid storage groove (1510); and
wherein the air backflow groove (153) laterally cuts the first fins (151) and at least some of the second fins (152) along a direction being parallel to an axis of the air-liquid equilibrium element (150), and communicates the first liquid storage grooves (1510) and at least some of the second liquid storage (1520) grooves with the liquid storage cavity (141).

12. The atomizer (100) according to any one of claims 1-8, wherein the air-liquid equilibrium element (150) further comprises a central through hole (1560), the atomizer (100) further comprises a liquid guide element (160) inserting in the central through hole (1560), the liquid guide element (160) fluidly connects the atomization assembly (100) with the liquid storage cavity (141).

13. The atomizer according to claim 12, wherein the air-liquid equilibrium element (150) further comprises a through groove (1562) communicating at least some of the plurality of liquid storage grooves (1510, 1520) with the central through hole (1560).

14. The atomizer (100, 200, 300) according to any one of claims 1-8, wherein the atomizer (100, 200, 300) further comprises a housing (110, 210, 310) connected with the base (120, 220, 320), the atomization assembly (130, 230, 330) is mounted on the base (120, 220, 320), the base (120, 220, 320) comprises an atomizing cavity (121, 2527) corresponding to the atomization assembly (130, 230, 330), the housing (110, 210, 310) comprises a gap (1131) communicated with the atomizing cavity (121, 2527); the atomizing cavity (121, 2527) and the gap (1131) form a part of the air flow passage; and
wherein the atomization assembly (130, 230, 330) comprises a porous ceramic substrate (131) mounted on the base (120, 220, 320) and a heating element (132) disposed on the porous ceramic substrate (131), the porous ceramic substrate (131) comprises a liquid absorption surface and an atomizing surface, the liquid absorption surface is connected with a lower end of the liquid guide element (160), the heating element (132) is mounted on the atomizing surface, and the atomizing surface corresponds to the atomizing cavity (121, 2527).

15. An electronic atomizing device, **characterized by** comprising an atomizer (100, 200, 300) according to any one of claims 1-14.
